# EUROPEAN PATENT APPLICATION

(11) **EP 1 029 914 A2**
(43) Date of publication of application: **23.08.2000**
(21) Application number: 00102170.8
(22) Date of filing: 09.02.2000
(51) Int. Cl.: C12N 15/12, C07K 14/47, C12Q 1/68, C07K 16/18

(54) **Growth hormone-regulated growth marker gene**

(30) Priority: 16.02.1999 JP 3761799
(71) Applicant: JCR PHARMACEUTICALS CO., LTD., Ashiya, Hyogo 659-0021 (JP)
(72) Inventor: Koga, Junichi, Hyogo (JP); Kono, Keiko, Hyogo (JP); Zolotaryov, Fyodor N., Hyogo (JP)
(74) Representative: Reinhard - Skuhra - Weise & Partner

(57) **Abstract**

A novel gene whose expression is regulated by human growth hormone, the DNA composing the gene, and the protein coded by the gene are disclosed. The DNA has a nucleotide sequence set forth under SEQ ID NO:1 in the Sequence Listing, and the protein coded by the gene has an amino acid sequence set forth under SEQ ID NO:2 in the Sequence Listing.

## Description

### FIELD OF THE INVENTION

The present invention relates to a gene whose expression is regulated by human growth hormone, a DNA composing the gene and a protein coded by the DNA, as well as diagnostic agents utilizing them.

### BACKGROUND OF THE INVENTION

Due to its growth stimulating effect, human growth hormone (hGH) has been widely used in the world as a therapeutic agent for children with growth hormone (GH) secretion deficiency, with Turner syndrome or with chronic nephropathy [Neely E.K.,and Rosenfeld R.G.,Ann.Rev.Med. 45:407-420 (1994)]. Having a wide variety of physiological activities, GH regulates the tissue-specific expression of a number of genes involved in growth, metabolism, differentiation, etc. Some of the effects of GH on cell growth or gene expression are gender-dependent or regulated by the GH secretion pattern from the pituitary. Growth factors including GH bind to respective receptors on cell membrane, which leads to phosphorylation of STAT (Signal Transducer and Activator of Transcription) in the signal transduction pathway of the cell, thereby transmitting the respective information to the nucleus. The effect of GH on the nuclear genes, however, has not been elucidated [Garry B. Udy, et al., Proc. Natl. Acad. Sci. U.S.A., 94: 7239-7244 (1997)].

Recently, since cardiac dysfunction was noted in patients with insufficient or excess secretion of GH, studies have been carried out about the effect of GH or insulin-like growth factor-1 (IGF-1) on the function and structure of the heart. It has been suggested, in particular, that GH or IGF-1 is involved in the regulation of development of the cardiac functions [Gaetano Lombardi et al., Journal of Pediatric Endocrinology & Metabolism, 10: 553-560 (1997)]. It has also been confirmed that the disorder of the cardiac functions may be partly restored by normalizing the levels of GH or IGF-1.

Meanwhile, a new gene was cloned from chromosome 21q22.3, which codes for a glutamic acid-rich protein and it has been confirmed that the expression of the gene takes place exclusively in the heart and skeletal muscles of adults [Paolo Scartezzini et al., Hum. Genet., 99: 387-392 (1997)].

Upon the above background, the objective of the present invention is to provide a new gene whose expression is regulated by human growth hormone, the DNA composing the gene, and the protein coded by the gene.

The present inventors found in studies using rabbit chondrocytes that there is a gene whose expression is regulated by growth factors such as growth hormone (GH), that the its expression is elevated in aged chondrocytes compared with younger ones, and that the elevated expression is restored by treatment with growth hormone to its expression levels observed in younger chondrocytes, and further that the gene is strongly expressed in abnormal cells including tumor cells. The inventors isolated, identified and analyzed the gene, and thereby revealed that it is a novel gene and it retains a common sequence of amino acids beyond species such as human and rabbit. The present invention was accomplished upon these findings.

### SUMMARY OF THE INVENTION

Thus, the present invention provides a DNA having a nucleotide sequence set forth under SEQ ID NO: 1 in the Sequence Listing.

The present invention further provides a protein having an amino acid sequence set forth under SEQ ID NO:2 in the Sequence Listing.

The present invention further provides a protein whose expression is enhanced in human tumor cells compared with human normal cells and consisting of an amino acid sequence with deletion, substitution or addition of one or more amino acids relative to the amino acid sequence set forth under SEQ ID NO:2 of the Sequence Listing. The examples of human tumor cells herein include promyelocytic leukemia cells, chronic myelogenous leukemia cells, colorectal adenocarcinoma cells, lung carcinoma cells and/or melanoma cells.

The present invention further provides the above-described proteins whose levels of expression are enhanced with aging and, by treatment with growth hormone, restored to the expression levels observed in younger cells.

The present invention still further provides DNAs each having a nucleotide sequence coding for one of above-described proteins. Such sequences can be constructed as desired with the aid of the common knowledge about the degeneracy of the genetic code.

The present invention further provides plasmids having ability of expressing one of the above-described DNAs.

The present invention further provides oligonucleotides specifically hybridizing to one of the above-described DNAs. Such oligonucleotides can be utilized for providing diagnostic agents in which they are employed as a primer, a probe or a marker.

In addition, the present invention also provides polyclonal or monoclonal antibodies each directed to one of the above-described proteins. The antibodies can be utilized for providing diagnostic agents based on immunoassay employing them.

### DETAILED DESCRIPTION OF THE INVENTION

The present inventors carried out a preliminary detection of genes regulated by growth hormone using the methods of mRNA differential display and PCR-select cDNA subtraction, and confirmed that there are genes in chondrocytes of rabbit whose expression are regulated by growth hormone: i.e., using mRNA differentiation display, an electrophoretic band (N 11) was found to be stimulated after 1-day treatment of rabbit chondrocytes with 100 ng/ml rhGH. This band was excised, its DNA extracted, cloned and sequenced by conventional methods. The length of this DNA fragment was found to be 505 n.b., and the DNA revealed no meaningful homology to any of the identified genes. However, there were found homologous human and mouse ESTs (Expression Sequence Tags). The DNA from this band was assigned homology cluster HCD20, and the respective rabbit gene was named "HCD20(11)".

Additional experiments revealed that the rabbit that had been used in mRNA differential display and thereby contributed to the discovery of HCD20(11), was somewhat abnormal, for we failed to detect any change in the expression of HCD20(11) gene by treatment with growth hormone in other young rabbits' chondrocytes. Surprisingly, however, it was found that the expression level of HCD20(11) gene was greatly elevated in aged, old cells and that treatment with GH restored it to lower levels characteristic of young rabbit chondrocytes. The full-length DNA sequence of HCD20(11) gene was determined using cDNA libraries that had been constructed with mRNA extracted from rabbit chondrocytes.

Since the nucleotide sequence of the 505 n.b. DNA fragment corresponding to this gene was shown to be highly homologous to a human EST as mentioned above, this rabbit HCD20(11)-specific DNA fragment was used as probe to search for a human analog of the gene (hGHRGM: human growth hormone regulated growth marker). The DNA fragment has the nucleotide sequence set forth under SEQ ID NO:3 in the Sequence Listing. Nine independent phage clones carrying various regions of hGHRGM gene were recovered. Their DNAs were purified and recloned into pUC18 vector and sequenced. Thus, a nucleotide sequence of a full-length of 765 bases was assembled. The sequence is set forth under SEQ ID NO:1 of the Sequence Listing. The deduced amino acid sequence of the putative protein consisting of 93 amino acid residues is also set forth under SEQ ID NO:2 of the Sequence Listing.

This protein is expected to be localized to the nucleus. It is shown to have very high conservatism beyond species since our determination has revealed no difference from the amino acid sequence of its rabbit analog. This suggests that the protein plays a role in fundamental functions of life.

The amino acid sequence of hGHRGM protein was compared to those of known polypeptides using BLASTP search. Human Scr homology 3H domain binding glutamic acid-rich-like protein (SH3BGRL) [Egeo A. et al., Biochem. Biophys. Res. Commun., 247(2): 302-306 (1998)] and human SH3 domain binding glutamic acid-rich protein (SH3BGR) [Paolo Scatezzini, et al., Hum. Genet., 99: 387-392 (1997)] showed the highest homology to hGHRGM protein. MOTIF search unveiled three motifs.

The proline-rich amino acid sequences in SH3BGR and SH3BGRL are known to play an important role in the protein-protein interaction associated with the signal transduction pathway [Cohen, G.B., et al., Cell, 214: 237-248 (1995). Powson, T. and Scott, J.D., Science, 278: 2075-2080 (1997)]. Specific, proline-rich common sequences bind to SH3. WW and EVH1 domains [Cicchetti, P. et al., Science, 257: 803-806 (1995). Macias, M.J. et al., Nature, 382: 646-649 (1996). Niebuhr, K. et al., EMBO J., 16: 5433-5444 (1997)]. Therefore, the compound of the present invention having such a proline-rich sequence is considered to take part in the signal transduction that gives rise to physiological functions of GH and IGF-I.

The transcript mRNA of the DNA of the present invention, the expression of which was noticed in chondrocytes through treatment with GH, was found strongly expressed in abnormal cells such as tumor cells. In addition, the expression of that mRNA in aged cells, which had been greatly elevated compared with younger cells, returned to the levels seen in younger cells after the aged cells were treated with GH. Recently, attention has been drawn to the treatment of adult patients having GH secretion deficiency by administering GH pharmaceutical preparations. The DNA, the protein coded by the DNA, antibodies directed to the protein, probes, etc. of the present invention may be utilized as diagnostic agents in such a treatment.

In addition, the determination of the expression levels of the protein of the present invention will be useful in monitoring the treatment with GH of children having GH secretion deficiency. The DNA, the protein coded by the DNA, antibodies directed to the protein, probes, etc. of the present invention, therefore, are also useful as diagnostic agents for such a purpose.

The present invention includes not only the DNA having the nucleotide sequence set forth under SEQ ID NO:1 in the Sequence Listing, but also DNAs composing a gene with similar properties and having deletion, substitution or addition of one or more nucleotides relative to that nucleotide sequence. The term "one or more nucleotides" generally means a few (e.g., 3 or 4) to 10 nucleotide bases, for example. The present invention also includes not only the protein having the amino acid sequence set forth under SEQ ID NO:2 in the Sequence Listing, but also proteins with similar properties and having deletion, substitution or addition of one or more amino adds relative to the amino acid sequence of that protein. The term "one or more amino acids" generally means a few (e.g., 3 or 4) to 10 amino acids. The present invention further includes DNA sequences coding such proteins. A variety of such nucleotide sequences can be readily prepared by those skilled in the art making use of the familiar knowledge on degeneracy of the genetic code.

A variety of mutants are available using recombinant DNA technology. First, a mutation can be introduced into a DNA clone fragment through different chemical and/or enzymatic processes, and the mutant DNAs thus obtained are then sequenced to select particular mutants with intended merits. This method allows a systematic preparation of different mutants regardless of their phenotypes. General methods of preparing a mutant clone DNA are as follows:
1. With the help of an oligonucleotide, substitution, deletion, insertion or addition can be directly carried out in a given DNA sequence. This method enables to introduce a number of mutations in a small region of a DNA.
2. Using a longer oligonucleotide, it is possible to synthesize a desired gene.
3. By means of region-specific mutagenesis, a desired mutation can be introduced into a large (1 - 3 kb) DNA region.
4. Linker-scanning mutagenesis of DNA is a method suited for introducing a cluster point mutation into a relatively small (4 - 10 bp) DNA region
5. PCR is also utilized as a method for direct introduction of a mutation.

### [References: Current Protocols in Molecular Biology., 3 Vols., Edited by Ausubel F.M. et al., John Wiley & Sons, Inc., Current Protocols., Vol. 1, Chapter 8: Mutagenesis of Cloned DNA, pages 8.0.1-8.5.10]

Also well known to those skilled in the art are methods of preparing plasmids and vectors which can express a desired gene including different mutations obtained by the above methods. That is, by inserting a DNA carrying a desired gene into a expression vector DNA using a combination of restriction enzymes and a ligase, a recombinant plasmid is readily constructed which carries the desired gene. The recombinant plasmid thus obtained is then introduced into different cells to transfect them, thereby producing transformed cells. Cells which may be utilized range from prokaryotes, e.g. E. coli, to yeast, insect, plant and animal cells.

### [References: Vectors Essential Data. Gacesa P. and Ramji D.P., 166 pages. BIOS Scientific Publishers Limited 1994., John Wiley & Sons in association with BIOS Scientific Publishers Ltd. Expression vectors, pages 9-12.]

Introduction of a recombinant plasmid into host cells is effected by calcium chloride method or electroporation. Calcium chloride method provides efficient transformation and requires no special apparatus. For higher efficiency, electroporation is recommended.

### [References: Current Protocols in Molecular Biology, 3 Vols. Edited by Ausbel F.M. et al., John Wiley & Sons, Inc., Current Protocols, Vol. 1, unit 1.8: Introduction of Plasmid DNA into Cells, pages 1.8.1-1.8.10]

Two types are known of transfection generally carried out on animal cell lines, i.e., transient and permanent types. In transient transfection, transformed cells are cultured for 1 - 4 days to effect transcription and replication of the transfected gene, and then the cells are harvested and their DNA analyzed. Alternatively, in many studies, a stable transformant cell line is produced, in which the transfected gene is incorporated into the chromosomes. Examples of the method for transfection include calcium phosphate method, electroporation, and liposome fusion method.

### [Reference: Current protocols in molecular biology. 3 vols. Edited by Ausubel F.M. et al., John Wiley & Son,Inc., Current Protocols. Vol.1, chapter 9: Introduction of DNA into mammalian cells, pages 9.0.1-9.17.3.]

Polyclonal and monoclonal antibodies directed to the proteins (polypeptides) coded by the gene of the present invention or their fragments and analogues as well, are readily prepared using techniques well known in the art. Antibodies thus obtained may be used as laboratory reagents and diagnostic agents for diseases associated with the gene of the present invention. The antibodies obtained are also used for preparation of antibody columns, for immunoprecipitation as well as for identification of the antigen by Western blotting.

A general method for preparing a monoclonal antibody in mg-scale directed to the proteins coded for by the gene of the present invention is as follows: Mice are inoculated with the antigen protein to immunize, and the spleen is removed from the mice exhibiting a sufficient antibody titer. The spleen cells are separated, and selected B cells are fused with myeloma cells of B cell origin to form hybridoma cells which secrete the antibody. The monoclonal antibody secreted from the hybridoma cells is purified from the culture medium by means of an affinity column, ion-exchange, or gel filtration, etc. The polyclonal antibody of the present invention also may be prepared by a conventional method: Using rabbits, horses, mice or guinea pigs as immunized animals, the antigen protein is inoculated along one of the schedules known in the art to immunize the animals, and then IgG, etc. are isolated from the collected serum.

### [Reference: Current protocols in molecular biology, 3 vols. Edited by Ausubel F.M. et al., John Wiley & Sons, Inc., Current Protocols, Vol. 2, chapter 11: Immunology, pages 11.0.1-11.16.13.]

The present inventors measured the expression levels of hGHRGM in a variety of human tissues using Northern hybridization. As it was found to occur in each of the tissues, hGHRGM is considered to be commonly expressed in various tissues. Its expression levels were compared between normal and tumor cells using a hGHRGM specific probe prepared. As a result, in comparison with normal cells, higher expression was found in tumor cells such as promyelocytic leukemia (HL-60), chronic myelogenous leukemia (K-562), colorectal adenocarcinoma (SW 480), lung carcinoma (A549), and melanoma (G631). This indicates that hGHRGM can be utilized as a diagnostic agent for assessing the stage of progression of certain tumors.

### EXAMPLES

The present invention is described in further detail with reference to the example below. However, it is not intended that the scope of the present invention be restricted to the example.

### 〈Preparation of Primers〉

The syntheses of M13 universal sequencing primer consisting of the nucleotide sequence set forth under SEQ ID NO:4 of the Sequence Listing and M13 reverse sequence primer consisting of the nucleotide sequence set forth under SEQ ID NO:5 in the Sequence Listing, both used for DNA sequencing of insertion in pUC18 vector, were ordered to Nisshinbo Tokyo Research Center.

### 〈Preparation of Phage Plating Cells〉

To obtain a high efficiency of phage infection, it is important that the cell used for phage growth are harvested in the logarithmic growth phase and are substantially free of dead cells. The XL1-Blue MRF' strain was found to be the most convenient to use. Its genotype is: Δ (mcrA)183 Δ (mcrCB-hsdSMR-mrr)173 endA1 supE44 thi-1 recA1 gyrA96 relA1 lac[F'proAB lacl^{q}Z Δ M15 Tn10(Tet^{r})]. The strain was cultured on CG agar plate (1.6 % Bacto-Agar (Difco) and 40 Capsules/L of CircleGrow (Bio 101, Inc.)) supplemented with 25 µg/ml tetracycline. The cells were incubated at 37°C and restreaked onto a fresh plate every fortnight, incubated overnight at 37°C and stored at 4°C.

The day before the plating stage, a single colony was picked from the CG agar plate, inoculated into 3 ml of CG broth (40 Capsules/L of CircleGrow) supplemented with 0.4% maltose and 25 µg/ml tetracycline, and incubated with shaking at 37°C overnight.

The 1.0 ml of the overnight culture was added to 47 ml of the same prewarmed growth medium and incubated at 37°C with vigorous shaking for 3 hrs. The cell density was estimated by spectrophotometry using a Beckman DU 640 spectrophotometer equipped with 0.1 ml quartz glass cuvettes. The magnitude of OD₆₀₀ is usually around 0.3 (1.5× 10⁸ cells/ml). The culture was transferred to sterile 50-ml Polycarbonate Centrifuge Bottles with Caps (Beckman) and spun at 3,000 rpm for 10 min at 4°C in a Beckman J2-MC Centrifuge using a Beckman JA 20 rotor. The cell pellet was resuspended in ice-cold sterile 10 mM MgSO₄ to prepare 2 × 10⁹ cells/ml suspension. The cells were placed on ice and used immediately for titration and plating of phages for screening.

### 〈Titration of Phages〉

One hundred-mm Petri dishes (Falcon) containing CG agar supplemented with 25 µg/ml tetracycline were used for titration. The Petri dishes in which the agar was poured were allowed to stand with their lids open at room temperature for 20 min to solidify the agar, then dried for 30 min by blowing sterile air at them in a safety cabinet (SCV-1303EC II B: HITACHI) and stored at 4°C. Before use, they were taken out of the refrigerator and warmed at 37°C for 1 hr in a constant-temperature room.

Two µl of phage mixture was mixed with 198 µl of SM buffer (100 mM NaCl, 8 mM MgSO₄, 0.01 % gelatin (SIGMA) and 50 mM Tris-HCl, pH 7.5). This dilution was labeled 10⁴ since, when 10 µl of the dilution was plated, a single plaque on the plate was equivalent to 10⁴ pfu/ml in the original phage stock. The successive dilutions down to 10⁹ were prepared in the same manner using SM buffer.

Ten µl of each of the phage dilutions was diluted with 90 µl of SM buffer, mixed with 100 µl of the cell suspension prepared in 10 mM MgCl₂ and incubated at 37°C for 15 min in a metal holder from a Thermo Alumi Bath ALB-120.

CG top agar (0.8% Type I-B Agarose (SIGMA), 40 Capsules/L of CircleGrow) was melted in a microwave stove and supplemented with 20% maltose up to 0.4% final concentration. Three-ml aliquots of the top agarose were added to a 16× 125 mm Tissue Culture Tube with a screw cap (FALCON) and kept before use at 48°C in the Thermo Alumi Bath AB-120.

Each of the plating compositions was added to the aforementioned tube containing melted agarose, mixed quickly by inverting the tube 5 times, and poured onto a 100-mm dry CG agar plate supplemented with 25 µ g/ml tetracycline. The top agar was allowed to set completely for 3 minutes before moving the plates. The plates were inverted and incubated at 37°C for 5 hrs and then overnight at room temperature. The number of the plaques was counted and the phage titer was calculated by multiplying the total number of plaques by the corresponding dilution number.

### 〈Plating of Phages and Transfer of Plaques onto Membranes〉

The plating protocol was basically the same as described above in association with the titration of phages. Up to 12 plates were processed at one time. The cDNA library was constructed with λ gt10 cloning vector by oligo(dT) + random priming method using a lung from a 75-year-old Caucasian male who had died because of trauma (Clontech).

For the first screening, 100 µ l of the phage library diluted with SM buffer (containing 50,000 - 500,000 pfu (plaque forming units)) was mixed with an equal volume of cells suspended in 10 mM MgCl₂, incubated for 15 min at 37°C, added to 7 ml of CG top agar at 48°C and mixed well by 5-times inversions, and poured onto a CG agar plate (150 mm: Falcon) supplemented with 25 µ g/ml tetracycline. The plates were inverted and incubated at 37°C for 5 hrs, and then overnight at room temperature.

Hybond^{TM}-N+ (a round, 132-mm, gridded, positively charged nylon membrane (Amersham)) was placed on the plaque-containing agar plate. The orientation of the filter was marked at three points with a 21G needle, and then the filter was removed and placed with its face up on the table and dried for at least 3 mm. For DNA fixation, the filter was placed on 2 sheets of Whatman paper saturated with 0.4 M NaOH for 10 - 30 min. To remove bacterial debris and alkali, the membrane was thoroughly washed twice with 5 × SSPE buffer (20 × SSPE: 3.0 M NaCl, 0.2 M NaH₂PO₄, 0.02 M EDTA, pH 7.4 (GibcoBRL)) and once with water, blotted with a paper towel and dried at room temperature.

### 〈Labeling and Purification of Probes〉

Multiprime DNA labeling system (Amersham) was used for labeling probes. Each of the four samples with 1 µ l of probe solution containing approximately 25 ng of a rabbit HCD20(11) specific DNA fragment was mixed with 26 µl of water, heated for 5 min at 98°C in the Thermo Alumi Bath ALB-120, and then chilled on ice. The samples were centrifuged at 18,000 × g (15,000 rpm on TOMY MRY- 150 High Speed Micro Refrigerated Centrifuge) for 1 sec to pull down the contents of the tubes. Each of the reaction mixtures was set up on ice in a MicroAmp Reaction Tube With Cap as follows: the total volume of the denatured DNA probe, 10 µ l of Labeling buffer, 5 µ l of Primer/BSA solution, 6 µ l of [α -³²P]dCTP (∼110 TBq/mmol, 370 MBq/ml) (Amersham) and 2 µl of Kienow enzyme. The blends were mixed gently by pipetting up and down, and incubated in a GeneAmp PCR System 2400 at 37°C for 30 min, kept at 20°C for 3 - 4 hrs until removal of unincorporated nucleotides and then hybridization.

QIAquick Nucleotide Removal Kit (Qiagen) was used for purification of the labeled probes. Each of the labeling mixtures was mixed with 500 µl of Buffer PN in a 1.5-ml tube. A QIAquick spin column was placed in a provided 2-ml collection tube. To allow binding of the DNA, the sample was applied to the QIAquick spin column and centrifuged at 6,000 rpm for 1 min (TOMY High Speed Micro Centrifuge MC-150). The QIAquick column was placed in a clean 2-ml collection tube, and the radioactive flow-through was retained for subsequent calculation of the label incorporation into the DNA. To wash QIAquick column, 500 µl of Buffer PE was added, and centrifugation carried out at 6,000 rpm for 1 min. The flow-through was discarded, and the washing repeated with another 500 µ l of Buffer PE. The flow-through was discarded, and the QIAquick column was placed back in the same tube. This tube should have been empty because residual ethanol from Buffer PE will not be completely removed unless the flow-through has been discarded before this additional centrifugation. The column was centrifuged for another 1 min at 13,000 rpm. The QIAquick column was placed in a clean 1.5-ml micro centrifuge tube. For elution of the DNA, 100 µl of Buffer EB (10 mM Tris-HCl, pH8.5) was added to the center of the column, and the column was allowed to stand for 1 min, and then centrifuged at 13,000 rpm for 1 min.

Each of the eluted labelled DNAs was transferred into a MicroAmp Reaction Tube With Cap, heated in a GeneAmp PCR System 2400 at 99.9°C for 5 min and chilled to 4°C. The denatured probe was added to a 1.5-ml tube containing 450 µ l of a hybridization buffer, and the efficiency of label incorporation was evaluated using a β(γ) Survey Meter TGS-133 (Aloka) and compared with the radioactivity of the flow-through nucleotides.

### 〈Plaque Hybridization〉

Hybridization and filter washing were performed at low temperatures because it was beneficial for precise positioning of filters to excise positive plaques. One hundred ml of hybridization buffer was prepared as follows: 50 ml of Formamide, deionized, Nuclease and Protease tested (Nacalai Tesque), 25 ml of 20 × SSPE Buffer (3M NaCl, 0.2 M NaH₂PO₄, 20 mM EDTA, pH 7.4)(GibcoBRL), 5 ml of 10 % SDS and 18 ml of water. Two 1.5-ml tube each containing 200 µl of 10 mg/ml Herring Sperm DNA Solution (GibcoBRL) and 0.8 ml of water were heated at 98°C in the Thermo Alumi Bath ALB-120 for 5 min, chilled on ice, and merged with the hybridization buffer. As SDS was prone to precipitate with decreasing temperature, the hybridization buffer was warmed to about 40°C just before use (in winter).

Eighty ml of the hybridization buffer was poured into a 150-mm round plastic container and 12 filters were immersed one by one taking care so that the front face of each membrane touches with the front face of an adjacent membrane and the back face of each membrane with the back face of an adjacent membrane. The lid was closed and the prehybridization was performed in a Thomastat Shaker T-22S at 32°C, at 40 rpm for at least 3 hrs.

The hybridization buffer was poured out from the prehybridized filters into an alternative 150-mm round plastic container, mixed with the remainder of the hybridization buffer and with the four labeled probes, and the filters were immersed as described above. The lid of the container was closed and the hybridization was effected in the Thomastat Shaker T-22S at 32°C and at 40 rpm overnight.

The filters placed in another round plastic container with a lid were washed by shaking in the Thomastat Shaker T-22S at 40 rpm with 250 ml each of the following washes:

| | | | |
|---|---|---|---|
| 1) | 2 × SSPE Buffer, 0.1 % SDS | 32°C | 10 min |
| 2) | 2 × SSPE Buffer, 0.1 % SDS | 50°C | 1.5 hrs |
| 3) | 0.5 × SSPE Buffer, 0.1 % SDS | 50°C | 1.5 hrs |

The residual washing buffer was blotted from the filters, which then were allowed to dry at room temperature for at least 1.5 hrs. Twelve filters were arranged in two 35.6 × 43.2-cm Fuji EC-A Cassettes and exposed to Hyperfilm-MP X-ray film (Amersham) for 3 - 4 days.

### 〈Recovery of Phages〉

The X-ray films were developed and the positions of the positive plaques were determined according to the orientation marks with the aid of a Bright Light Box for illumination. The 5 × 5-mm top agarose plugs, with their centers coincided with positive signals on the X-ray films, were excised with 21G needles and put into 4-ml sample vials with caps each containing 0.3 ml of SM buffer. The phages were eluted for 1 day at 4°C.

### 〈Second Screening of Phages〉

The procedures of the second screening of phages was basically the same as described above for the first screening. After preliminary adjustment, no more titration of the phage was required for plating. An amount of phages corresponding to 0.0001 µ l of the phage eluate per 150-mm Petri dish was found to be, in most cases, appropriate. The individual plaques were eluted with 0.2 ml of SM buffer at 4°C for 1 day, supplemented with 14 µl of DMSO (dimethyl sulfoxide), and stored at -70 °C until use in propagation and purification of the phage DNA.

### 〈Preparation of Phage DNA〉

In the morning of the day before the plating stage, a single colony was picked up from the CG agar plate, inoculated into 3 ml of CG broth (40 Capsules/L of CircleGrow) supplemented with 0.4 % maltose and 25 µ g/ml tetracycline, and incubated at 37°C with shaking. In the evening of the same day, 50 µl of the culture was added to 47 ml of the same pre-warmed growth medium and incubated at 37°C overnight with vigorous shaking. In the morning of the following day, the culture was transferred to a sterile 50-ml Polycarbonate Centrifuge Bottle With Cap (Beckman) and spun at 3,000 rpm for 10 min at 4°C in a Beckman J2-MC Centrifuge using a Beckman JA 20 rotor. The cell pellet thus obtained was resuspended in 4.5 ml of chilled sterile 10 mM MgSO₄, and then placed on ice.

The 150-mm Petri dishes (Falcon) containing 1.2 % CG agarose GP-36 supplemented with 25 µ g/ml tetracycline were used for propagation of the phage. The Petri dishes with poured agar were not dried after solidification. They were stored in a refrigerator, and warmed for 1 hr at 37°C before use in a constant-temperature room.

Fifty µl of phage eluate containing approximately 5 × 10⁶ pfu was mixed well with 450 µl of SM buffer and 500 µl of the cell suspension, incubated at 37°C for 15 min, mixed with 6 ml of melted agarose and spread onto a Petri dish. Three minutes later, the Petri dishes were inverted and incubated at 37°C for approximately 8 hrs.

To elute the phages, each of the plates was covered with 8 ml of SM buffer and 200 µl of chloroform, fastened to a Rotary Shaker R-20 mini (Taitec), and shaken at 150 rpm overnight at 4°C.

The phage eluate was transferred into a 15-ml conical centrifuge tube (Greiner) and the plate was washed with additional 2 ml of SM buffer for 1 hr at room temperature. The second eluate was combined with the first one and the tube was spun at 3,500 rpm for 10 min (TOMY TS-7 rotor: Low Speed Centrifuge TOMY LC-122).

QIAGEN Lambda Mini Kit (QIAGEN) was used for purification of the phage DNA. Eight ml of cleared plate lysate was transferred to a new 15-ml conical centrifuge tube, mixed with 25 µl of Buffer LI (300 mM NaCl, 100 mM Tris-HCl, pH 7.5, 10 mM EDTA, 0.2 mg/ml BSA, 20 mg/ml RNase A and 6 mg/ml DNase I) and incubated at 37°C for 30 min in the Thermo Alumi Bath ALB-120 (Iwaki). The lysate was mixed with 1.6 ml of ice-cold Buffer L2 (30 % polyethylene glycol PEG 6000, 3 M NaCl) and incubated on ice for 60 min. The content of the tube was transferred into a 16 × 76-mm centrifuge tube (Beckman) and centrifuged in the 50 Ti rotor of a L70 Ultracentrifuge (Beckman) for 15 min at 15,000 rpm at 4°C. The supernate was discarded, and the tube was placed upside down for 1 min to allow the residual fluid to drain. The phage precipitate formed by the PEG precipitation carried out in the previous step was hardly visible as it was clear and spread on the wall of the tube. Therefore, 0.65 ml of Buffer L3 (100 mM NaCl, 100 mM Tris-HCl, pH 7.5, 25 mM EDTA) was pipetted several times over the wall to ensure complete resuspension of the pellet, and then the content was transferred into a 2-ml safe-lock tube (Eppendorf). An equal volume of Buffer 4 (4 % sodium dodecyl sulfate) was added to the tube, mixed gently, heated at 70°C for 10 min in the Thermo Alumi Bath ALB-120, then cooled on ice. A volume of 0.65 ml of Buffer LS (3 M potassium acetate, pH 5.5) were poured into the tube, mixed immediately but gently and spun at 4°C for 30 min at 15,000 rpm (High Speed Micro Refrigerated Centrifuge MRX-150 (TOMY)). The supernate was transferred to a new 2-ml tube and centrifuged again for 10 min at 15,000 rpm at room temperature to remove any residual suspended materials.

While the centrifugation was going on, a QIAGEN-tip 20 was placed in a QIAtrack 1 over the waste tray, and the column was equilibrated with 1 ml of Buffer QBT (750 mM NaCl, 50 mM MOPS, pH 7.0, 15 % ethanol, 0.15 % Triton X-100). The QIAGEN-tip was allowed to drain completely. QIAGEN-tip was able to be left unattended since the resin bed retained some buffer and would not readily dry out.

The supernate was promptly applied onto the QIAGEN-tip and allowed to flow through the resin by gravity. The tip was washed twice with 1 ml of Buffer QC (1.0 M NaCl, 50 mM MOPS, pH 7.0, 15 % ethanol). The upper part of a QIArack 1 was placed over the lower rack fitted with clean 1.5-ml tubes and the DNA was eluted twice with 0.75 ml of Buffer QC (1.25 M NaCl, 50 mM Tris-HCl, pH 8.5, 15 % ethanol) into two alternating 1.5-ml micro centrifuge tubes (Treff Lab).

The DNA was precipitated with 0.7 volumes of isopropanol and centrifuged for 30 min at 15,000 rpm at room temperature. The supernate was carefully removed and discarded. The DNA pellet was briefly washed with 0.5 ml of 70 % ethanol at room-temperature, and then recentrifuged. This washing with 70 % ethanol at room-temperature was repeated. Ethanol was completely removed. The pellet was briefly air-dried for 5 min, the DNA in each tube was dissolved in 18 µl of NaOH (pH 8) and the content of two tubes containing the same DNA were combined.

### 〈Recloning of Phage Insertions〉

To determine the sequences of the phage insertions, the DNA fragments from appropriate clones were recloned into pUC18 vector.

36 µl of λDNA solution was mixed with 4.5 µl of 10×H buffer and 4.5 µ l of EcoRI (10 U / µl) (Takara). Digestion proceeded overnight at 37°C, then the mixture was distributed among three wells and electrophoresed using 1 % Agarose GP-36 gel at 50 V for 50 min. The proper bands were excised, and gel pieces carrying DNA fragments from the same phage clone were combined.

The insertions were purified using a QIAEX II Gel Extraction Kit. For DNA fragments of 100 bp - 4 kb, three volumes of Buffer QX1 were added to one volume of the gel. QIAEX II was resuspended by vortexing for 30 sec, and 10 µl of the resin was added to each of the samples. The mixtures were incubated at 50 °C for 12 min in the Thermo Alumi Bath ALB-120 (Iwaki).

The samples were briefly vortexed every 2 min. The tubes were centrifuged at 18,000 × g (15,000 rpm on TOMY MRX-150 High Speed Micro Refrigerated Centrifuge) for 1 min and the supernates were discarded. Five hundred µl of Buffer QX1 was added to each of the DNA-containing pellets, briefly vortexed, centrifuged at 18,000 × g for 1 min and the supernate was completely removed. Five hundred µl of Buffer PE was added to each of the DNA-containing pellets, briefly vortexed, centrifuged at 18,000 × g for 1 min and the supernate was completely removed. This washing step was repeated one more time. The pellets were air-dried for 25 - 30 min until they turned white. Vacuum drying was avoided because over-drying of the pellets would cause lower recoveries. Purified water was added to each of the samples, the pellets were resuspended by vortexing and incubated at room temperature for 5 min with occasional vortexing. The samples were centrifuged at 18,000 × g for 1 min and the DNA-containing supernates were transferred to clean tubes. This step was repeated with incubating the mixture at 50 °C. The first and the second DNA eluates of the same DNA fragment were combined. Purified DNA fragments were ligated immediately with vector DNA or stored at -20 °C.

Five µl of fragment-containing eluate was mixed with 15 µl of water and added to the tube of Ready-To-Go pUC18 EcoRI/BAP+Ligase (Pharmacia Biotech). The mixture was incubated at room temperature for 3 min, then at 16°C for 0.5 - 3 hrs in a MIR-153 incubator (Sanyo).

One tube containing 100 µl of DH5 Competent High Cells (Toyobo) was thawed and stirred to obtain evenly suspended cells. Ten to twenty-five µl of the competent cells were pipetted into each of the prechilled 1.5-ml tubes. One fiftieth of the ligation mixture was added directly to the cells and stirred gently to mix. All the cell were used at one time, for critical loss of transformation efficiency had been noted following an additional cycle of freezing and thawing. The tubes were left to stand on ice for 30 min. The cells were heat-shocked for exactly 45 sec at 42°C in the Thermo Alumi Bath ALB-120 and placed on ice for 30 sec. Ten volumes of SOC medium at room temperature were added to each of the tubes. The tubes were left to stand at 37°C for 1 hr. One tenth of the transformants were spread on the plate containing 1.4 % Bacto-Agar (Difco), 40 Capsules/L of CircleGrow (Bio 101, Inc.) and 100 µg/ml of ampicillin. The plates were incubated at 37°C overnight.

### 〈Purification of Plasmid DNAs〉

Two colonies were picked from each of the plates containing the transformants using sterile toothpicks. The bacteria were transferred to 14-ml polypropylene tubes with caps (Falcon) containing 2.5 ml of CircleGrow medium (40 Capsules/L) and 100 µg/ml of ampicillin. The tubes were shaken at 200 rpm for 16 - 72 hrs at 37°C (1 day cultivation gives the maximal yield of plasmid DNA).

RPM AFS Kit (Bio 101, Inc.) was employed for rapid isolation and purification of double-stranded DNAs from the bacterial cultures. Up to 12 plasmid DNAs were purified at one time. The bacterial cells were spun in 2-ml Safe-Lock Tubes (Eppendorf) at 18,000×g (15,000 rpm on TOMY MRX-150 High Speed Micro Refrigerated Centrifuge) for 1 min. The supernates were decanted and discarded. The cells were resuspended in 1 ml water by vortexing, and spun again. The supernates were decanted and discarded. Each of the cell pellets was resuspended in 200 µl of Pre-Lysis Buffer #1 by vortexing until the cells were completely resuspended. Four hundred µl of Alkaline Lysis Solution #2 was added directly to the cell suspensions, and the tubes were gently inverted 15 times. One min later, 300 µl of ice-cold Neutralizing Solution #3 was added, and the tubes were shaken vigorously 3 - 5 times until a uniform white precipitate formed. The mixtures were incubated for 5 min on ice, spun for 5 min at room temperature, and the supernates were transferred to new 2-ml tubes.

Five hundred µ l of Glassmilk SpinBuffer #4 was added to the supernates, the mixture turned up and down for 5 min for effecting efficient binding of the DNA to the Glassmilk, and spun at 18,000 × g for 2 sec. The supernates were decanted and discarded. Each of the Glassmilk/DNA complex was resuspended in 500 µl of Wash Solution #5 by gently stirring with a pipette tip and then by pipetting up and down, and transferred to a kit-supplied RPM AFS Spin Filter. The filter was spun for 10 sec to lower the level of the wash solution to the level of the pellet without drying it. The Catch Tube was emptied, and the Spin Filter was reassembled. Five hundred µl of Wash Solution was added to each of the Spin Filters, followed by centrifugation for 2 min to "dry" the filter content. The spin filters were transferred to new kit-supplied RPM AFS Catch Tubes. One hundred and forty µ l of Elution Solution #8 (RNase/DNase/pyrogen free H₂O) was added to each of the samples, and the Glassmilk/DNA complex was mixed to a slurry by gentle finger tapping. The plasmid DNAs were collected in the Catch Tubes by centrifugation at 15,000 rpm for 3 min. The DNA solutions were stored at -20 °C.

### 〈Detection of DNA Inserts〉

The presence of insertions and appropriate amount of DNA solution for DNA sequencing were determined by agarose electrophoresis of plasmid DNAs digested with restriction endonucleases. Two µl each of plasmid DNA solutions was mixed with 11.3 µl of H₂O, 1.5 µl of 10×H Universal Buffer (0.5 M Tris-HCl, pH 7.5, 100mM MgCl₂, 10mM DTT, 1 M NaCl), 0.2 µl of EcoRI (10 U/µl) (purchased from Takara), and incubated at 37°C for at least 2 hrs.

Separation gel was prepared by melting, in a microwave oven, the mixture containing 1 % Agarose GP-36 (Nacalai Tesque), 0.5 µ g/ml Ethidium Bromide (Sigma) and 0.5 × TBE buffer, pouring the thus obtained solution into the mould of Mupid-2 Mini-Gel Electrophoresis System (Advance), and allowing to solidify at room temperature for at least 30 min.

Total volume of each of the digested plasmid DNA was mixed with 1.5 µl of 10 × Loading buffer (50 % glycerol, 0.01 % bromphenol blue) and applied to the gel. pHY marker (Takara) was used as a standard marker for DNA molecular weight. Gel electrophoreses were run at 100 V for 30 min using a Mupid-2 Mini-Gel Electrophoresis System containing 0.5 × TBE buffer in the buffer chambers.

The pictures were produced using FAS-II (Toyobo): the gels were illuminated with an Electronic U.V. Transilluminator, the pictures were taken with a XC-75/75CE CCD Video Camera Module (Sony) and printed with a Video Graphic Printer UP-880 (Sony). DNAs from clones carrying insertions with proper lengths were chosen for DNA sequencing.

### 〈Preparation of Samples for DNA Sequencing〉

ABI PRISM™ Dye Terminator Cycle Sequencing Ready Reaction Kit with AmpliTaq DNA Polymerase, FS was employed for DNA sequencing. Up to 18 PCRs were performed at one time. The reaction mixtures were prepared in MicroAmp Reaction Tubes With Caps (Perkin Elmer) as follows: eight µ l of Terminator Ready Reaction Mix, 2 µl of M13 Universal Sequencing Primer or M13 Reverse Sequence Primer (2 µ M), 0.1 - 0.5 µg of plasmid DNA (volume of the DNA solution required was estimated based on the electrophoretic pictures) and H₂O up to a final volume of 20 µl. The blends were pipetted up and down to mix well. PCRs were carried out in a GeneAmp PCR System 9600 thermal cycler (Perkin Elmer) and according to the following amplification cycles:

### [25 Cycles Repeated]

- 10 sec:: 96 °C (denaturation)
- 5 sec:: 50 °C (annealing)
- 4 min:: 60 °C (extension)

### [Final step]

- indefinitely: 4 °C (hold)

The PCR products were purified using ethanol precipitation. For each reaction product, a 1.5-ml microcentrifuge tube was prepared added with 2 µl of 3 M sodium acetate (pH 5.2) and 50 µl of 99.5 % ethanol. The entire 20-µl content of each reaction tube was transferred to the microcentrifuge tube containing the ethanol solution. The tubes were vortexed and placed on ice for 10 min. The mixtures then were centrifuged at 18,000×g (15,000 rpm on TOMY MC-150 High Speed Micro Centrifuge) for 20 min at 4 °C. The ethanol solution was carefully aspirated with a micropipette. The pellets were rinsed by addition of 250 µl of 70 % ethanol and centrifuged at 15,000 rpm for 1 min. The alcohol solution was aspirated with a micropipette carefully to avoid disturbing the pellets. The tubes were dried under vacuum for 10 min. The dried precipitates were used immediately or stored at -20 °C.

### 〈DNA Sequencing〉

A 373-18 DNA Sequencer (Applied Biosystems) was employed for sequencing the plasmid DNAs. Since the cleanness of glassware is highly important for credible and accurate sequencing, the glassware was carefully washed with water and then wiped with isopropyl alcohol. The 4.75 % denaturing PAAG containing 8.3 M urea was used for separation of the PCR products: 19.94 g of urea was mixed in a 50-ml centrifuge tube with 4.75 ml of 40 % (19:1) Acrylamide/Bis Solution (Bio-Rad Laboratories) and 8 ml of 5 ×TBE Buffer, and distilled water was added to make 40 ml. The tube was vigorously stirred, warmed in a microwave stove for one turn of its dish, and vigorously stirred again until all urea crystals were dissolved. The solution was degassed in a desiccator equipped with an A-3S aspirator while the gel casting equipment was being prepared (approximately 10 min). Nineteen µl of TEMED (Sigma) and 170 µl of 10 % ammonium persulfate (Sigma) (stored at -20°C) were added, gently mixed for 30 sec and poured into the glass plate mould (420 × 250 × 0.25 mm). The gel was allowed to solidify at room temperature for 2 - 5 hrs

A loading buffer was prepared by mixing 52 µl of formamide with 10 µl of 3 % blue dextran (Sigma) in 50 mM EDTA (pH 8.0). The sequencing samples were dissolved in 3 µl of this buffer, heated at 94°C for 2 min in the Thermo Alumi Bath ALB-120 (Iwaki) and then kept on ice. The glass plates with the gel were carefully washed once more, checked by scanning, and the electrophoresis chambers were assembled. The shark teeth comb was inserted at 1 mm dip, 1 × TBE buffer was poured into the both buffer chambers, and pre-electrophoresis performed for 20 - 30 min. PMT voltage was set at 900 V and run parameters were set at 2500 V, 20 mA and 30 W. The wells were carefully washed with 1 × TBE buffer using a syringe, and the samples were loaded in the odd wells, and electrophoresed for 9 hrs. The wells were washed once again, and the samples were loaded in the even wells and electrophoresed for 9 hrs. The Data Collection computer program was started immediately after the electrophoresis was run.

ANALYSIS computer program was activated automatically after the completion of data collection. Software's mistakes were corrected manually. The initial analysis of the nucleotide sequences was performed using DNASIS software.

### 〈Construction of Nested Deletions〉

Controlled digestion of the DNA with exonuclease III was employed for constructing unidirectional deletion in double-stranded DNA for sequencing of the recloned DNA insertions. This enzyme is a 3'-exonuclease which is active only on double-stranded DNA. Blunt and 5'-overhanging ends are susceptible to digestion with the enzyme, while 3'-overhanging ends made of three or more basis are resistant to it. In addition, since phosphorothioate bonds in the phosphate backbone are resistant to digestion with exonuclease III, recessed 3'-ends (5'-overhangs) which have been "filled-in" with thionucleotides are also resistant to digestion. At least two independent clones carrying each DNA region of the gene were used with Double-Stranded Nested Deletion Kit (Pharmacia Biotech) to create deletions from opposite ends.

Appropriate restriction endonucleases must not cut the DNA insertions. If a suitable pair of restriction enzymes could be utilized which produce 3'-and 5'-overhangs or blunt-end, then 2 µ g of the purified plasmid DNA was digested with these enzymes for at least 3 hrs. Two-µl aliquots were used to monitor the progress of the digestion by agarose gel electrophoresis. When both digestions were completed, the DNA sample was heated for 10 min at 70°C to inactivate the enzymes.

End-protection by thionucleotides was chosen if no appropriate pair of restriction enzymes could be found and neither endonuclease produced 3'-overhanging ends. In such a case, 2 µ g of the plasmid DNA solution was digested with a first restriction endonuclease in a volume of 10 µl. Up to 3 µl of this reaction mixture was used to monitor the progress of the reaction. Diluted Klenow fragment (0.05 units/ µ l) was prepared by mixing FPLCpure Kienow Fragment (1 unit/ µl) with 20 µl of 1 ×Klenow Buffer (50 mM Tris-HCl, pH 7.5; 10 mM MgCl₂ and 0.1 mM DTT). To a 1.5-ml microcentrifuge tube was added the following: 7 µl of restriction-digested DNA, 1 µl of 10 × Klenow Buffer, 1 µl of dNTPαS Mix (aqueous solution containing 400 µ M each of dATPαS, dCTPαS, dGTPαS and dTTPαS) and 1 µ l of Diluted Klenow Fragment. The tube was gently mixed, briefly centrifuged and incubated at 37°C for 15 min. The reaction mixture was heated at 65°C for 20 min, followed by the addition of 20 µl of NaCl/glycogen (aqueous solution containing 250 mM NaCl and 250 ng/ µl glycogen) and 75 µl of ethanol. The mixture was placed on dry ice for 10 min or cooled at -80 for at least 1 hr. The precipitated DNA was collected by centrifugation at 4°C for 10 min at 15,000 rpm. The supernate was carefully removed and discarded. The pellet was rinsed with 250 µl of 70 % ethanol and centrifuged for 1 min. The supernate was carefully removed. The pellet was dried under vacuum for 5 min and redissolved in 10 µl of water. The DNA was digested with the second restriction enzyme in a final reaction volume of 20 µl, and heated at 70°C for 10 min to inactivate the enzyme.

In a microcentrifuge tube, a S1 nuclease/buffer mixture was prepared as follows: 33 µl of S1 Buffer, 66 µl of distilled water and 1 µl of S1 nuclease. Three µl of this mixture was pipetted into each of 20 microcentrifuge tubes and placed on ice.

Twenty-four µl of 2 × ExoIII buffer with appropriate NaCl concentration was prepared. The concentration of NaCl should be 75 mM or lower after dilution with an equal volume of the double-digested DNA.

In a 1.5-ml microcentrifuge tube, 20 µl of 2 × ExoIII buffer was mixed with 20 µl (2 µg) of the double-digested DNA, and equilibrated at 30°C for 2 - 3 min in the Thermo Alumi Bath ALB-120. An aliquot of 2 µl was removed as the "time = 0" control sample, and placed in an appropriate tube containing 3 µl of S1 nuclease/buffer, mixed well and put on ice.

1 µ l of Exonuclease III was added, mixed gently, and incubation was continued at 30°C. A 2-µl sample was removed from the reaction mixture every 5 min, immediately and thoroughly mixed with 3 µl of S1 nuclease/buffer. All these tubes were kept on ice until all the timed samples were removed from the exonuclease III reaction mixture.

After all of the timed samples were taken and mixed with S1 nuclease/buffer, they were incubated simultaneously at room temperature for 30 min.

One µl of S1 Nuclease Stop Solution was added to each of the samples, and the tubes were incubated at 65°C for 30 min. Half of each timed sample was used for electrophoretic analysis of deletions, and the remaining half was used for recircularization by ligation. These two processes were carried out simultaneously for all samples, and the results of the deletion analysis were then used to select recircularized samples to be used for transformation.

Three µl of each of the samples was mixed with 2 µl of Loading Buffer (50 % glycerol, 1 mM EDTA and 0.01 % bromphenol blue) and analyzed by 30-min electrophoresis on a 1 % agarose gel (Agarose GP-36: Nacalai Tesqu) containing 0.5 µg/ml of ethidium bromide.

While the electrophoresis was in progress, the remaining 3 µl of each of the timed samples was used for re-ligation. A ligation mixture was prepared by mixing the following materials in a 1.5-ml microcentrifuge tube: 40 µl of 10× Ligation Buffer, 80 µl of 25 % PEG, 2 µl of T4 DNA ligase and 218 µl of distilled water. Seventeen µl of this ligation mixtures was added to the 3 µl of each of the timed samples, mixed gently and incubated at room temperature for 2 hrs.

When the electrophoresis was completed, the gel was inspected to determine which timed samples contained deletions of interest. Just those samples that contained deletions of interest were used to transform E. coli cells. Two µl of the ligation reaction mixture was employed for transformation of 10 µ l of DH5 Competent High Cells (Toyobo). Transformations were accomplished according to "Recloning of Phage Insertions" protocol. All the transformed cells were spread on plates each containing 1.4 % Bacto-Agar (Difco), 40 Capsules/L of CircleGrow (Bio 101, Inc.) and 100 µ g/ml ampicillin. The plates were incubated at 37°C overnight. Two colonies from each plate were grown and analyzed for the extent of deletions by electrophoresis of the plasmid DNAs digested with EcoRI followed by DNA sequencing as described above. The DNA sequences were linked with DNASIS computer program to compile the full-length insertion.

### 〈Computer Analysis of Sequencing Data〉

The analysis of nucleotide sequences was performed using DNASIS computer program and the following software available through the Internet: BLAST (Basic Local Alignment Search Tool), MOTIF (Protein Sequence Motif Search), PSORT (Prediction of Protein Sorting Signals and Localization Sites in Amino Acid Sequences), SOSUI (Prediction of Transmembrane Segments), SIM (Alignment Tool for protein sequences) and GeneStream align.

### 〈Northern Hybridization〉

Multiple Tissue Northern (MTN) Blots (Clontech) containing approximately 2 µ g of polyA⁺RNA per lane from different human cells were hybridized with rabbit HCD20(11)-specific DNA probes as described above in "Plaque Hybridization". BIOMAX MS scientific imaging film (Kodak) was employed since it is nearly eight times more sensitive than Hyperfilm-MP. The film was exposed to the membranes using Fuji EC-A Cassette with intensifying screen at -80°C for 3 weeks. Before development of the film, the cassette was warmed for at least 1 hr at room temperature.

## Claims

1. A DNA having a nucleotide sequence set forth under SEQ ID NO:1 in the Sequence Listing.

2. A protein having an amino acid sequence set forth under SEQ ID NO:2 in the Sequence Listing.

3. A protein whose expression is enhanced in human tumor cells compared with human normal cells and consisting of an amino acid sequence with deletion, substitution or addition of one or more amino acids relative to the amino acid sequence recited in claim 2.

4. The protein of claim 3 wherein said human tumor cells are promyelocytic leukemia cells, chronic myelogenous leukemia cells, colorectal adenocarcinoma cells, lung carcinoma cells and/or melanoma cells.

5. A protein whose expression is enhanced with aging and, by treatment with growth hormone, restored to the expression levels observed in younger cells and consisting of an amino acid sequence with deletion, substitution or addition of one or more amino acids relative to the amino acid sequence recited in claim 2.

6. A protein of claim 3 whose expression is enhanced with aging and, by treatment with growth hormone, restored to the expression levels observed in younger cells.

7. A protein of claim 4 whose expression is enhanced with aging and, by treatment with growth hormone, restored to the expression levels observed in younger cells.

8. A DNA having a nucleotide sequence coding for a protein of one of claims 2 to 7.

9. A plasmid having ability of expressing a DNA of claim 1.

10. A plasmid having ability of expressing a DNA of claim 8.

11. An oligonucleotide which can specifically hybridize to a DNA of claim 1.

12. An oligonucleotide which can specifically hybridize to a DNA of claim 8.

13. A diagnostic agent utilizing the oligonucleotide of claim 1 as a primer, a probe or a marker.

14. A diagnostic agent utilizing the oligonucleotide of claim 8 as a primer, a probe or a marker.

15. An antibody directed to the protein of one of the claims 2 to 7.

16. A diagnostic agent utilizing the antibody of claim 15.


SEQUENCE LISTING

<110> JCR Pharmaceuticals Co., Ltd.

<120> Growth hormone-regulated growth marker

<130> GP20

<160> 5

<210> 1
<211> 765
<212> DNA
<213> Homo sapiens

<400> 1
agcacggcgg cggcgtcgtc tcccggcagt gcagctgccg ctaccgccgc cctctgcccg     60

ccggcccgtc tgtctacccc cagc atg agc ggc ctg cgc gtc tac agc acg     111
                           Met Ser Gly Leu Arg Val Tyr Ser Thr
                             1               5

tcg gtc acc ggc tcc cgc gaa atc aag tcc cag cag agc gag gtg acc     159
Ser Val Thr Gly Ser Arg Glu Ile Lys Ser Gln Gln Ser Glu Val Thr
 10                  15                  20                  25

cga atc ctg gat ggg aag cgc atc caa tac cag cta gtg gac atc tcc     207
Arg Ile Leu Asp Gly Lys Arg Ile Gln Tyr Gln Leu Val Asp Ile Ser
                 30                  35                  40

cag gac aac gcc ctg agg gat gag atg cga gcc ttg gca ggc aac ccc     255
Gln Asp Asn Ala Leu Arg Asp Glu Met Arg Ala Leu Ala Gly Asn Pro
             45                  50                  55

aag gcc acc cca ccc cag att gtc aac ggg gac cag tac tgt ggg gac 303
Lys Ala Thr Pro Pro Gln Ile Val Asn Gly Asp Gln Tyr Cys Gly Asp
        60                  65                  70

tat gag ctc ttc gtg gag gct gtg gaa caa aac acg ctg cag gag ttc 351
Tyr Glu Leu Phe Val Glu Ala Val Glu Gln Asn Thr Leu Gln Glu Phe
    75                  80                  85

ctg aag ctg gct tgagtcaagc ctgtccagag ttcccctgct ggactccatc 403
Leu Lys Leu Ala
90

accacactcc ccccagcctt cacctggcca tgaaggacct tttgaccaac tccctgtcat 463

tcctaaccta accttagagt ccctccccca atgcaggcca cttctcctcc ctcctctcta 523

aatgtagtcc cctctcctcc atctaaaggc aacattcctt acccattagt ctcagaaatt 583

gtcttaagca acagccccaa atgctggctg cccccagcca agcattgggg ccgccatcct 643

gcctggcact ggctgatggg cacctctgtt ggttccatca gccagagctc tgccaaaggc 703

cccgcagtcc ctctcccagg aggaccctag aggcaattaa atgatgtcct gttccattgg 763

cg 765

<210> 2
<211> 93
<212> PRT
<213> Homo sapiens

<400> 2
Met Ser Gly Leu Arg Val Tyr Ser Thr Ser Val Thr Gly Ser Arg Glu

1                   5                   10                  15
Ile Lys Ser Gln Gln Ser Glu Val Thr Arg Ile Leu Asp Gly Lys Arg
            20                  25                  30

Ile Gln Tyr Gln Leu Val Asp Ile Ser Gln Asp Asn Ala Leu Arg Asp
        35                  40                  45

Glu Met Arg Ala Leu Ala Gly Asn Pro Lys Ala Thr Pro Pro Gln Ile
    50                  55                  60

Val Asn Gly Asp Gln Tyr Cys Gly Asp Tyr Glu Leu Phe Val Glu Ala
65                  70                  75                  80

Val Glu Gln Asn Thr Leu Gln Glu Phe Leu Lys Leu Ala
                85                  90

<210> 3
<211> 505
<212> DNA
<213> Oryctolagus cuniculus

<400> 3
gttttttttt ttttttcagt ggaatgggca tttaattgtc tagggtgctc ccaggcaagg    60

gactgcgggc ctttggcaga gctctggctc aggaaccaag aagaggtgtc ctggtcagtg    120

ccaggcagag aggcggcagc cccaaggccg ggctgggacg gccagcactg gggctgctgc    180

ttaagacatt tcggagacgg agcggagaag aggcttggcc tggggagagg gtccccaagg    240

ccaaaaggtt aggaatgaca gggaagttgt cacaagtccc ttcgaggcca gttgaggcct    300

gcggggagaa catgtgaccg ggtccagggg aacccatcgg gggctggact cagccagctt    360

caggaactcc tgcagcgtgt tttgctccac agcctccacg aagagctcat agtccccgca 420

gtattggtcc ccgttgacga tctggggtgg gtgccttggg gttgcccgcc aaggctcgca 480

tctcatcccg cagggcgttg tcctg 505

<210> 4
<211> 17
<212> DNA
<213> M13

<400> 4
gtaaaacgac ggccagt 17

<210> 5
<211> 17
<212> DNA
<213> M13

<400> 5
caggaaacag ctatgac 17